# EUROPEAN PATENT APPLICATION

(11) **EP 4 282 425 A1**
(43) Date of publication of application: **29.11.2023**
(21) Application number: 22741964.5
(22) Date of filing: 07.01.2022
(51) Int. Cl.: A61K 38/07, A61P 3/10, A61P 7/02, A61P 9/10, A61P 9/12, A61P 13/12, A61P 37/02, A61P 31/14

(54) **USE OF COMPOUND, PHARMACEUTICAL COMPOSITION FOR THE TREATMENT OF IMMUNE OR METABOLIC DISORDERS, PHARMACEUTICAL COMPOSITION FOR THE TREATMENT OF ILLNESSES CAUSED BY OR ASSOCIATED WITH VIRUSES**

(30) Priority: 19.01.2021 BR 102021001035
(71) Applicant: Proteimax Biotecnologia Ltda, 05581001 São Paulo (BR); Remer Consultores Assessoria Empresarial Ltda., 20010-020 Rio De Janeiro (BR)
(72) Inventor: REMER, Ricardo Amaral, 22471-001 Rio De Janeiro (BR); HEIMANN, Andrea Sterman, 04531-000 São Paulo (BR)
(74) Representative: Grünecker Patent- und Rechtsanwälte PartG mbB
(86) International application number: PCT/BR2022/050007
(87) International publication number: WO 2022/155716

(57) **Abstract**

The present invention pertains to the fields of pharmaceutics, medicine, biotechnology and immunology. More specifically, the present invention describes the use of a compound or of a combination of compounds for preparing a pharmaceutical composition to reduce levels of TNFα, IL-6, and/or IL-1β. The invention can be used to treat immune or metabolic disorders associated with high levels of TNFα, IL-6, IL-1β, or with high levels of ACE-2 and the respective cardiac, renal and blood-pressure-related consequences, also including the problems associated with treatments involving antibodies, such as monoclonal antibodies, or cell therapies, such as CAR T-cell therapies, or conditions such as hypertension, myocarditis, pericarditis, coagulopathies, thrombotic, cardiovascular or renal events, or type-2 diabetes, and diseases caused by viruses such as influenza or coronavirus, including COVID-19. The in-vivo administration of the composition of the invention has surprising results on the modulation of cytokine levels in mammals, and said cytokines are related to high levels of ACE-2, have been shown to be correlated with the severity of the disease associated with SARS-CoV-2 (COVID-19), and have been identified as indicative markers of the prognosis of the severity thereof, and are also related to myocarditis, pericarditis, coagulopathies, thrombotic or cardiovascular events, and immune or metabolic disorders. Also disclosed are a pharmaceutical composition and a method for treating said conditions.

## Description

### Field of the Invention

The present invention lies in the fields of Pharmacy, Medicine, Biotechnology, and Immunology. More specifically, the present invention describes the use of a compound or a combination of compounds for the preparation of a pharmaceutical composition for reducing TNFα, IL-6, and/or IL-1β levels. The invention is useful for the treatment of immune or metabolic disorders associated with elevated levels of TNFα, IL-6, IL-1 β, or elevated levels of ACE2 and the related cardiac, renal and blood pressure consequences, also including problems associated with therapies involving antibodies, such as monoclonal, or cell therapies, such as CAR-T, or with conditions such as hypertension, myocarditis, pericarditis, coagulopathies, thrombotic, cardiovascular or renal events, or type 2 diabetes, and diseases caused by viruses such as influenza or coronavirus, including COVID-19. The in vivo administration of the composition of the invention provided surprising results in the modulation of cytokine levels in mammals, cytokines that are related to elevated levels of ACE2, have been shown to be correlated with the severity of the disease associated with SarsCov2 (COVID-19) and have been identified as indicative markers of the prognosis of its severity, and are also related to myocarditis, pericarditis, coagulopathies, thrombotic or cardiovascular events, and immune or metabolic disorders. Also disclosed is a pharmaceutical composition and a therapeutic method for treating these conditions.

### Background of the Invention

The peptide compound which use is the object of the invention is surprisingly stable in extremes of temperature, does not lead to the problem of fibril formation and provides easy manipulation in the preparation of products of pharmaceutical interest, including ligands of diagnostic and pharmaceutical composition interests.

The peptide compound used in the invention is the subject of co-pending patent application BR 11 2019 002655 0 (Brazilian National Phase of PCTBR2018 050156), incorporated herein by reference and which constitutes the closest prior art. Said compound is a synthetic peptide that has been studied by the present inventors in Brazil since 2017. The study derives from another study related to hemopressin (PVNFKFLSH), a nonapeptide discovered by a Brazilian team, which received this name because it is derived from hemoglobin and because it is hypotensive (*"*Novel Natural Peptide Substrates for Endopeptidase 24.15 Neurolysin, and Angiotensin-converting Enzyme"; Vanessa Rioli, Fabio C. Gozzo, Andrea S. Heimann, Alessandra Linardi, José E. Krieger, Claudio S. Shida, Paulo C. Almeida, Stephen Hyslop, Marcos N. Eberlin, Emer S. Ferro; March 7, 2003).

The peptide compound which use is the object of the invention has been shown, in previous patent applications by the same inventors (including WO 2018209415), as a useful improvement over hemopressin, as it is surprisingly more stable in extremes of temperature and for not leading to the problem of formation of fibrils. In addition, said compound also proved to be an improved ligand of cannabinoid receptors CB1 and CB2 when compared to hemopressin, with proven results in vivo, in vitro and in silico. (de Araujo CB, Heimann AS, Remer RA, Russo LC, Colquhoun A, Forti FL, et al. Intracellular Peptides in Cell Biology and Pharmacology. Biomolecules 2019;9(4); WO 2018209415). Said article does not mention anything about TNFα and makes no mention or suggestion of the use of said compound for the treatment of conditions associated with elevated levels of cytokines. The present invention surprisingly provides this new and important use.

The peptide compound which use is the object of the present invention also showed surprisingly contrasting results in relation to hemopressin: scientific studies suggest that hemopressin does not cross the blood-brain barrier, but it causes an anxiogenic effect (Pharmacol Biochem Behav, 2015 Feb;129:7-13 "Anxiogenic-like effects induced by hemopressin in rats"), probably via fragments thereof crossing the blood-brain barrier. Other study (Pharmacol Rep. 2017 Dec;69(6):1247-1253, "Emotional disorders induced by Hemopressin and RVD-hemopressin(α) administration in rats") demonstrated that the administration of hemopressin induced anxiogenic and depressive behavior, reduction in the basal levels of monoamine in the prefrontal cortex and increased expression of genes involved in its catabolism, in contrast to the extended version known as RVD-hemopressin, which administration induced anxiolytic and antidepressant behavior. The data shown in the present patent application show that the administration of the compound of the invention does not induce an undesirable response from the Central Nervous System, that is, it does not cause the known central effects in its closest congeners. This important technical advantage enables, in practice, its use in a wider range of conditions.

Elevated levels of pro-inflammatory cytokines are associated with a variety of conditions or disorders in mammals, notably cytokine release syndrome or cytokine storm.

In this context, the problem caused by some immunotherapies with antibodies, such as monoclonal ones, and cell therapies, such as CAR-T, is well known.

What happened in 2006, during clinical trials of the antibody TGN-1412 (theralizumab) - which caused severe inflammatory reactions - led to changes in the approach to developing and testing therapies with antibody, which now include specific tests for the release of pro-inflammatory cytokines that can trigger the so-called cytokine release syndrome.

Antibodies and monoclonal antibodies are widely used in various treatments. Although effective, they can cause adverse effects related to cytokine release or cytokine release syndrome. The present invention provides an alternative to reduce or eliminate these undesirable effects.

A common approach to assessing the risk of cytokine release is to incubate cells with a pro-inflammatory inducer such as anti-CD3 antibody. The present invention uses this inducer as an experimental model and demonstrates that the administration of the compound of the invention reduces the levels of inflammatory cytokines even when the animal is stimulated with anti-CD3.

The article entitled "CAR-T cell-induced cytokine release syndrome is mediated by macrophages and ablated by IL-1 blockade" (Nat. Med 2018 Jun 24(6):731-738) shows that the decrease in IL-1 levels minimizes the immune disturbance associated with said therapy.

A similar approach is revealed in the article entitled "Therapeutic potential of TNFα and IL-1β blockade for CRS/ICANS in CAR-T therapy via ameliorating endothelial activation" (Front. Immunol, 2021 May 19 (12)623610). Said article shows that the decrease in TNFα and IL-1 β levels minimizes the immune disorder associated with said therapy. The present invention provides the decrease of TNFα and IL-1β levels.

Aberrant pulmonary immune responses are associated with several respiratory infections, including Influenza, with elevated cytokine levels associated with a cytokine storm being frequent in these cases. This condition is difficult to treat, and simple treatment with corticosteroids is generally not sufficient to treat the pathology associated with influenza and/or coronavirus infections. It is well known in severe or lethal cases of Influenza that the levels of TNFα and IL-6 are greatly increased, and that reducing the levels of these cytokines is important in reducing the severity of the disease. (Seo, SH; Hoffmann, E.; Webster, RG. Nature Medicine, Vol 8 No. 9, 2002 Lethal H5N1 influenza viruses escape host anti-viral cytokine responses). The present invention provides for lowering the levels of these and other cytokines.

The article published in the journal Biomedicines 2021, 9, 1576, available at https://doi.org/10.3390/biomedicines9111576A is entitled "Rapid and Simple Multiparameter Assay to Quantify Spike-Specific CD4 and CD8 T Cells after SARS-CoV-2 Vaccination: A Preliminary Report". Said article was published on 29Oct2021 and reveals that TNF-α is the main cytokine produced by memory T cells after in vitro stimulation with specific antigens of the coronavirus Spike protein and is also an important marker of T cell induction through vaccination. The present invention provides for lowering the levels of this and other cytokines.

The article by Hariharan A, Hakeem AR, Radhakrishnan S, Reddy MS, Rela M. "The Role and Therapeutic Potential of NF-kappa-B Pathway in Severe COVID-19 Patients", Inflammopharmacology Feb;29(1):91-100, published in Feb2021 reveals that COVID-19 involves overactivation of the NF-κB pathway. A similar report is made by Kircheis R, Haasbach E, Lueftenegger D, Heyken WT, Ocker M, Planz O. NF-κB Pathway as a Potential Target for Treatment of Critical Stage COVID-19 Patients, Front Immunol. Published in Dec2020.

In this context, the literature also demonstrated that the Spike protein of SarsCov-2 potentially induces the expression of the NF-κB pathway and inflammatory cytokines, including TNFα, IL-6, IL-1β in human and mouse macrophages (Khan S, Shafiei MS, Longoria C, Schoggins J, Savani RC, Zaki H. SARS-CoV-2 spike protein induces inflammation via TLR2-dependent activation of the NF-κB pathway, bioRxiv published in Mar2021).

The literature shows that not only SarsCov-2 and COVID-19 induce the expression of inflammatory cytokines such as TNFα, IL-6, IL-1β. Also, the Spike protein alone is capable of significantly inducing the expression of these cytokines. The present invention provides the decrease of TNFα, IL-6 and IL-1β levels.

According to clinical research results with the Spike recombinant protein vaccine demonstrate, TNFα levels were greatly increased following immunization. The research was published in the *New England Journal of Medicine* in Sep2020 (N Engl J Med 2020;383:2320-32), the article being titled "Phase 1-2 Trial of a SARS-CoV-2 Recombinant Spike Protein Nanoparticle Vaccine".

The article entitled "The SARS-CoV-2 Spike protein disrupts human cardiac pericytes function through CD147-receptor-mediated signalling: a potential non-infective mechanism of COVID-19 microvascular disease" is a preprint from bioRxiv (doi: https://doi.org/10.1101/2020.12.21.423721) made available on 20Jul2021. Said article reveals in detail the relationship of pericytes (essential perivascular cells in maintaining metabolic, mechanical and signaling functions in microvessels) with SarsCov-2 and with the Spike protein of the said virus. The article demonstrates that the virus does not infect pericytes in vitro, but that Spike protein alone (recombinant) causes profound changes in pericytes, including increased migration, reduced ability to support matrix/network formation in the endothelium, increased secretion of inflammatory cytokines typical of the cytokine storm and production of pro-apoptotic factors responsible for the death of endothelial cells. Said article shows that the levels of TNFα and IL-6 increase substantially with the presence of the Spike protein alone.

In the article published by the present inventors ("NFKF is a synthetic fragment derived from rat hemopressin that protects from neurodegeneration" Neuroscience Letters 721 (2020) 134765), it was demonstrated that the peptide compound used in the present invention is a good candidate for neuroprotection for oral use, having shown excellent results of neuroprotection in vivo. In addition to being a non-harmful publication under the terms of the law, said article does not reveal any information about the modulation of TNFα and does not include any mention or suggestion of its use in the treatment of diseases associated with viruses or coronaviruses, its modulation of ACE2, among others.

TNF-α and IL-6 have been shown to be important markers of the severity of COVID-19 in recent months. In this context, in October 2020, the article entitled "An inflammatory cytokine signature predicts COVID-19 severity and survival" (Nature Medicine, vol 26 pag. 1636-1643, Oct2020). This article shows that serum levels of IL-6 and TNF-α are significant predictors of the severity of the disease and the survival profile: the higher the levels of these cytokines, the worse the prognosis and the greater the severity of the disease. Consequently, finding therapeutic alternatives that act advantageously to decrease the level of one and/or the other, or even better, of both of these cytokines is very useful in the context of combating the severity of COVID-19, both for the potential treatment of patients in serious condition, as well as for potential early treatment or prophylaxis. The present invention provides an advantageous therapeutic alternative, which can also be administered orally, among other routes.

Studies from over a decade ago (J Interferon Cytokine Res. 2011 Oct; 31(10): 705-710. Critical Cytokine Pathways to Cardiac Inflammation) demonstrated that myocarditis events are clearly associated with the elevation of IL-1 β and TNF-α levels, indicating that the elevation of the level of these cytokines is necessary and sufficient to induce the occurrence of autoimmune diseases, specifically in the heart. In addition, said article showed that the severity of myocarditis is also related to the profile of these cytokines.

Consistent with the above finding, more recently, in the context of COVID-19, it was reported that severe COVID-19 with cytokine storm was successfully treated with Anakinra (a recombinant IL-1 receptor antagonist) with dexamethasone (CJC Open, Volume 3, Issue 2, February 2021, Pages 210-213; Rapid Response to Cytokine Storm Inhibition Using Anakinra in a Patient With COVID-19 Myocarditis). The patient, 62 years old and with a history of primary progressive Multiple Sclerosis, was diagnosed with COVID-19 and presented acute alteration of consciousness, hypoxia, and shock. Said treatment resulted in rapid clinical improvement and reduction in serum levels of inflammatory markers (TNFα, IL-6, IL-1), as well as memorable recovery of the heart assessed by nuclear magnetic resonance. The patient was discharged from the hospital after treatment.

In the article entitled "The Relationship between Inflammatory Cytokines and Coagulopathy in Patients with COVID-19", published on 09May2021 on J. Clin. Med. 2021, 10, 2020. https://doi.org/10.3390/jcm10092020, the correlation between the elevated profile of TNFα, IL-6 cytokines with an increased risk of developing coagulopathies, thrombotic events, and deaths during the course of COVID-19 is demonstrated. It also suggests that early measurement and rapid administration of anti-inflammatories with anticoagulants may decrease the incidence of thrombotic events and their fatal consequences. The results presented in this report show that the present invention provides a useful therapeutic alternative for preventive, prophylactic, early or curative treatment of coagulopathies, thrombotic and/or cardiovascular events.

The article published in the magazine Diabetes, Vol 64, issue 4, 01Apr2015: 1273-1283, "Angiotensin II Induces Interleukin-1β-Mediated Islet Inflammation and β-Cell Dysfunction Independently of Vasoconstrictive Effects", reveals that pathological activation of the renin-angiotensin system is associated with the metabolic syndrome, and that inhibition of the renin-angiotensin system can delay the onset of type 2 diabetes. Said article indicates that the modulation of the renin-angiotensin system, notably through the inhibition of the cytokine IL-1β, is a potential therapeutic approach for the treatment of patients with type 2 diabetes. The present invention, by providing a substantial reduction in IL-1β levels, is a therapeutic alternative for the preventive, prophylactic or curative treatment of type 2 diabetes.

The cytokines TNFα, IL-6 and IL-1β, in addition to all the implications indicated above, also play an important role in the regulation of the renin-angiotensin system complex and in the angiotensin-converting enzymes ACE and ACE2, which has important impacts on various conditions, such as hypertension, various cardiovascular and renal events, as well as in COVID-19, since ACE2 has a recognized role in the infection process by the respective virus.

On the one hand, it is known that TNFα alone (Hypertension, 2008 May 51(5): 1345-51, Involvement of tumor necrosis factor-alpha in angiotensin II-mediated effects on salt appetite, hypertension, and cardiac hypertrophy), as well as IL-6 alone (The Journal of Rheumatology doi: 10.3899/jrheum.200547 "Angiotensin-converting enzyme 2, a SarsCov-2 receptor, is upregulated by interlekin-6 via STAT3 signaling in rheumatoid synovium"), are able to increase ACE2 expression. Therefore, providing a therapeutic approach that concomitantly decreases TNFα and IL-6 levels is highly desirable to reduce ACE2 expression. Reducing ACE2 expression has a variety of therapeutic applications, including the treatment of hypertension, cardiovascular or renal events, as well as potentially limiting the progression of SarsCov-2 infection.

On the other hand, ACE2 increases the expression of IL-6 and IL1-β, creating positive feedback (increase in ACE2 due to IL-6) which may be one of the factors responsible for the cytokine storm or similar events and which is related to the severity of the disease COVID-19. Consequently, the present invention provides a therapeutic alternative that can contribute to the fight against COVID-19 by more than one different mechanism: contribute to minimize or prevent viral replication by reducing the expression of ACE2; and contribute to minimize the severe disease associated with the cytokine storm.

In addition, IL1-β and TNFα additively increase (i.e., inhibiting only one may not be sufficient in therapeutic approaches) the expression of angiotensin II receptor type AT1 in cardiac fibroblasts (Journal of Molecular and Cellular Cardiology 2005 Mar; 38(3):505-1; "IL-1 beta and TNF-alpha upregulate angiotensin II type 1 (AT(1)) receptors on cardiac fibroblasts and are associated with increased AT(1) density in the post-MI heart").

Therefore, providing a therapeutic approach that concomitantly decreases the levels of IL1-β and TNFα is highly desirable to reduce the expression of the angiotensin II receptor type AT1 in cardiac fibroblasts.

Finally, it is known that ACE regulates ACE2 expression, and that hemopressin, a potent hypotensive, is probably an ACE inhibitor, given the inhibition constant Ki (1866 µM) observed by the present co-inventor in an article published in 2003 (J Biol Chemistry Vol 278 No 10, 2003 pp 8547-8555; "Novel natural peptide substrates for endopeptidase 24.15, neurolysin, and angiotensin-converting enzyme*").

From what the most recent literature teaches, providing a pharmaceutical composition that is useful in reducing the levels of TNFα, IL-6 and/or IL-1β is highly desirable in the context of combating the effects of several conditions associated with elevated levels of inflammatory cytokines. Examples include: treatments with antibodies; cell therapies such as CAR-T; viral infections with influenza or coronavirus, such as COVID-19; myocarditis; pericarditis; coagulopathies; hypertension, thrombotic, cardiovascular or renal events; disorders associated with Spike protein, viruses and/or vaccine vectors and type 2 diabetes. The present invention does exactly this. The results presented in the present patent application also support the affirmation that the administration of the composition of the invention can interfere with the expression of ACE2, modulate the renin-angiotensin system and/or modulate the ACE/ACE2 balance.

From what can be deduced from the researched literature, no documents were found anticipating or suggesting the teachings of the present invention, much less its surprising technical effects, so that the solution proposed herein, in the eyes of the inventors, has novelty and inventive activity in view of the state of the art.

### Summary of the Invention

The present invention provides a new therapeutic alternative to substantially reduce the levels of: tumor necrosis factor alpha (TNFα), interleukin 6 (IL-6) and/or interleukin 1 beta (IL-1β) in mammals. Therefore, the present invention provides a new therapeutic alternative for the treatment of immune or metabolic disorders associated with elevated levels of one or more from these cytokines.

The present invention also provides a new therapeutic alternative to reduce the expression of ACE2 and/or modulate the ACE/ACE2 balance, being therefore useful in the treatment of hypertension, cardiovascular or renal events and potentially to reduce the process of viral infection by SarsCov-2.

The present invention also provides a new therapeutic alternative to reduce or eliminate the problems associated with therapies involving antibodies, such as monoclonal, or cell therapies, such as CAR-T, or conditions associated thereof.

The present invention also provides a new therapeutic alternative for the treatment of infections or diseases caused by or associated with viruses, such as influenza, coronavirus, such as SarsCov-2, and COVID-19, or conditions associated with these viruses or parts thereof.

It is an object of the invention to use a peptide compound to manufacture a medicament for reducing substantially the levels of TNFα, IL-6 and/or IL-1β in mammals, as a new therapeutic alternative for the treatment of immune or metabolic disorders associated with elevated levels of one or more from these cytokines. Said peptide compound is selected from NFKF, NFKL, and/or modified, cyclic forms thereof, amidated, alkylated, alkoxylated, halogenated, hydroxylated, PEGylated versions, modified with other functional groups, as well as with a non-natural amino acid such as d-amino acids forms, salts thereof; and/or combinations thereof.

It is another object of the invention to use said peptide compound to manufacture a preventive, curative or prophylactic medicament for infections or diseases caused by or associated with viruses such as influenza, coronavirus, such as SarsCov-2, and COVID-19, or of conditions associated with these viruses or parts thereof.

Another object of the invention is a pharmaceutical composition for the preventive, curative or prophylactic treatment of infections or diseases caused by or associated with viruses, such as influenza, coronaviruses, such as SarsCov-2, and COVID-19 or conditions associated with these viruses or parts thereof.

Another object of the invention is a pharmaceutical composition for the preventive, curative or prophylactic treatment of immune disorders or metabolic disorders associated with elevated levels of TNFα, IL-6 and/or IL-1β in mammals.

It is another object of the invention to provide a pharmaceutical composition for the preventive, curative or prophylactic treatment of hypertension, myocarditis and/or pericarditis.

It is another object of the invention to provide a pharmaceutical composition for the preventive, curative or prophylactic treatment of coagulopathies, thrombotic, cardiovascular or renal events, and type 2 diabetes.

It is another object of the invention to provide an alternative pharmaceutical composition to monoclonal antibodies being tested and/or used to reduce TNFα, IL-6 and/or IL-1β levels in the treatment of COVID-19 or side effects associated to virus or to the Spike protein.

The pharmaceutical composition of the invention comprises said peptide compound and a pharmaceutically acceptable vehicle and may be in the form of a pastille, tablet, gel, oral liquid or syrup, capsule, suppository, injectable solution or inhalable forms or patch, and may optionally comprise other active principles.

It is another object of the invention to provide a therapeutic method for treating the conditions indicated in this specification.

These and other objects of the present patent application will be immediately valued by those skilled in the art and by companies with interests in the segment and will be described in sufficient detail for their reproduction in the following description.

### Brief Description of Figures

The following detailed description and the attached figures illustrate the main characteristics and embodiments of the present invention, presented in detail to give better support to the person skilled in the art and so that they can understand and reproduce the inventive concept of the invention in any embodiment. Such details or figures should not be understood in a limiting manner and only serve to illustrate some of the embodiments of the present invention.

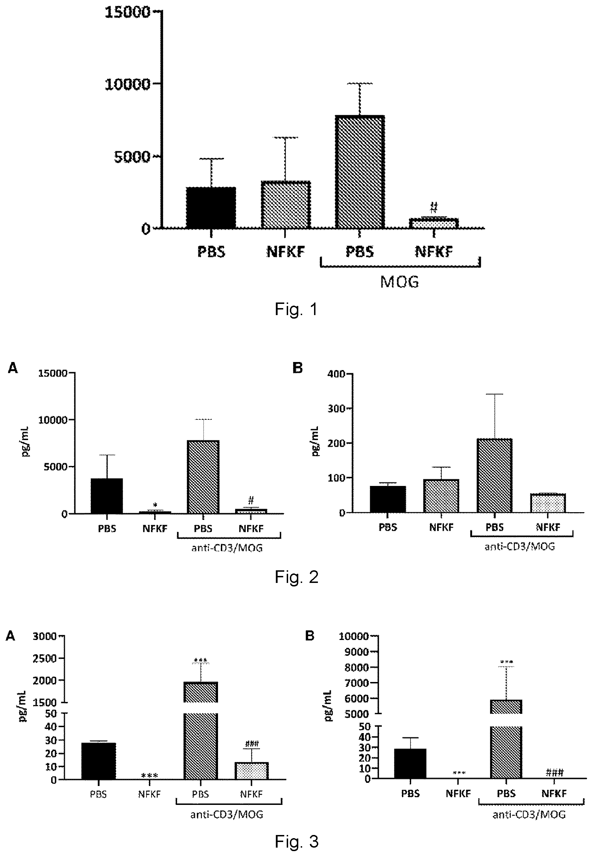

Fig. 1 shows the results of tests for the evaluation of TNFα levels and modulation thereof in an established experimental model, in which female C57BL6 animals (mice), aged 8 to 10 weeks old, were submitted to an Experimental Autoimmune Encephalomyelitis (EAE) induction protocol. On the y-axis, TNFα concentrations are shown in arbitrary units, under different test conditions as indicated, in which 10 µL of plasma were used for quantification by ELISA according to the manufacturer's specifications (R&D Systems). Results are expressed as mean ± standard deviation. Statistical analyzes were performed using the One-way ANOVA test, n = 4. # p<0.1 vs. MOG.
Fig. 2 shows the results of tests for the evaluation of the level of IL-6 and modulation thereof in an established experimental model, in which with females C57BL6 animals (mice), from 8 to 10 weeks old, which were submitted to an Experimental Autoimmune Encephalomyelitis (EAE) induction protocol. Eight days after EAE induction, the animals were anesthetized with ketamine (90 mg/kg) and xylazine (10 mg/kg). After dissection of the spleen and inguinal lymph nodes, leukocytes were extracted. Cells were stimulated with Anti-CD3 (1 µg/mL) and MOG (100 µg/mL) with or without said peptide compound in the NFKF embodiment (100 µg/mL) for 72 hours. Then, 50 µL of medium were used for IL-6 cytokine quantification by ELISA. In A, IL-6 is shown by quantification by ELISA on spleen leukocyte cells. In B, it is IL-6 as measured by ELISA in leukocyte cells from inguinal lymph nodes. Results are expressed as mean ± standard deviation. Statistical analyzes were performed using One-way ANOVA test, n = 4. * p<0.1 vs. PBS; # p<0.1 vs. CD3/MOG.
Fig. 3 shows the results of tests for the evaluation of the level of IL-1β and modulation thereof in an established experimental model, in which with females C57BL6 animals (mice), from 8 to 10 weeks old, which were submitted to an Experimental Autoimmune Encephalomyelitis (EAE) induction protocol. Eight days after EAE induction, the animals were anesthetized with ketamine (90 mg/kg) and xylazine (10 mg/kg). After dissection of the spleen and inguinal lymph nodes, leukocytes were extracted. Cells were stimulated with Anti-CD3 (1 µg/mL) and MOG (100 µg/mL) with or without said peptide compound in the NFKF embodiment (100 µg/mL) for 72 hours. Then, 50 µL of medium were used for IL-1β cytokine quantification by ELISA. In A, IL-1β is shown by quantification by ELISA in spleen leukocyte cells. In B, it is IL-1β as measured by ELISA in leukocyte cells from inguinal lymph nodes. Results are expressed as mean ± standard deviation. Statistical analyzes were performed using the One-way ANOVA test, n = 4. *** p<0.001 vs. PBS; ### p<0.001 vs. CD3/MOG.

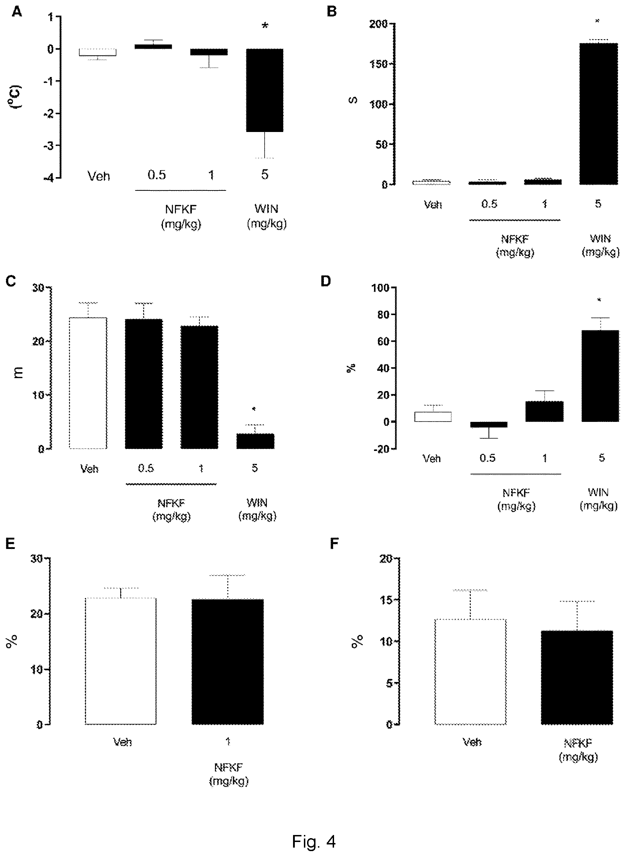

Fig. 4 shows the results of the elevated plus maze (EPM) test, using saline solution (Veh) as a negative control, the compound Win 55212-2 (WIN), a known cannabinoid receptor 1 agonist, as a positive control and NFKF at the indicated concentrations, all administered orally (by gavage) in C57B6 mice, in a total of 7 animals. In A) the temperature variation is shown in °C (delta °C); in B) the time of catalepsy in seconds after administration is shown; in C) the walking distance in meters is shown in the open field walking test; in D) the % of MPE (maximal possible effect) in the hot plate test is shown; in E) the % of entries in the open arms in the EPM (elevated plus maze) test is shown; in F) the % of time spent in the open arms in the EPM test is shown. Results are expressed as mean ± standard deviation. Statistical analyzes were performed using the One-way ANOVA test, n=7. * P<0.05.

### Detailed Description of the Invention

The invention provides a new use of the peptide compound selected from NFKF, NFKL, and/or modified, cyclic forms thereof, amidated, alkylated, alkoxylated, halogenated, hydroxylated, PEGylated versions, modified with other functional groups, as well as with a non-natural amino acid as d-amino acid forms, salts thereof; and/or combinations thereof.

In the present invention, said compound is used for the manufacture of a medicament useful for reducing the levels of cytokines such as tumor necrosis factor alpha (TNFα), interleukin 6 (IL-6) and/or interleukin 1 beta (IL-1β) in mammals.

In the present invention, said compound is useful for the manufacture of a preventive, curative or prophylactic medicament for immune disorders or metabolic disorders associated with elevated levels of TNFα, IL-6 and/or IL-1β in mammals. Examples include problems associated with therapies involving antibodies, such as monoclonal, or cell therapies, such as CAR-T, or conditions associated with the elevation of such cytokines, such as myocarditis, pericarditis, coagulopathies, thrombotic and/or cardiovascular events, and type 2 diabetes.

In the present invention, said compound is useful for the manufacture of a preventive, curative or prophylactic medicament for infections or diseases caused by or associated with viruses, such as influenza, coronavirus, such as SarsCov2, and COVID-19.

In the present invention, said compound is useful for the manufacture of a medicament for reducing ACE2 expression and/or modulating the ACE/ACE2 balance, thus being useful in the treatment of hypertension, cardiovascular or renal events and potentially for reducing the viral infection process by SarsCov-2.

The compound used in the invention is synthetic. Said peptide compound has surprisingly high stability in extremes of temperature, in addition to easy manipulation, being particularly useful in pharmaceutical and medicament preparations. The peptide compound which use is the object of the invention provides, among others, advantages in administration, bioavailability and/or therapeutic action in a mammal, when compared to other therapeutic agents with the same or similar purpose.

The invention is a very useful alternative as an adjunct to antibody therapies, monoclonal antibodies, or cell therapies, to reduce or eliminate the problems associated with these therapies.

The invention is also a very useful alternative for adjuvant or substitute therapy for monoclonal antibodies used to reduce the levels of TNFα, IL-6 and/or IL-1β, which are injectable and/or not very accessible, but which have shown important results in the treatment of COVID-19 or associated late effects or side effects. Examples of such monoclonal antibodies include adalimumab, infliximab, golilumab, certolizumab, sarilumab and tocilizumab.

In the examples shown throughout the present patent application, it is presented data and results of experiments that prove that the oral administration of the pharmaceutical composition containing said peptide compound provided substantial and vigorous reduction of the levels of TNFα, IL-6 and IL-1β in mammals.

For purposes of the present invention the following definitions are used:

### Product of pharmaceutical interest

In the context of the present patent application, "compound of pharmaceutical interest" should be understood as any molecular entity comprising the compound which use is the inventive concept common to the present patent application, also including molecular entities obtained through chemical modification/derivatization thereof, with the inclusion of other functional groups, linear or branched side chains, alteration of hydrophilicity or hydrophobicity, among others, provided that they comprise the NFKF, NFKL entity or variants thereof as defined above as their nucleus, except for natural and already known entities.

### Pharmaceutical composition

In the context of the present patent application, "pharmaceutical composition" should be understood as any and all compositions containing an active ingredient, with prophylactic, palliative and/or curative purposes, acting in such a way as to maintain and/or restore homeostasis, and may be administered orally, topically, parenterally, enterally, intrathecally, intranasally and/or pulmonarily.

### Pharmaceutically acceptable formulation

In the context of the present patent application, "pharmaceutically acceptable formulation" should be understood as a formulation containing pharmaceutically acceptable excipients and carriers well known to those skilled in the art, as well as the development of convenient doses and treatments for use in particular compositions that can be described in a range of treatment regimens, including oral, parenteral, intravenous, intranasal, pulmonary, intravitreal and intramuscular, intracerebral, intracerebroventricular and intraocular and administration and/or formulation thereof.

### Modified peptide

In the context of the present patent application, "modified peptide" should be understood as a non-natural peptide, artificially modified or obtained by synthesis, including halides, cyclized, amidated, alkylated, alkoxylated, hydroxylated, PEGylated, other functional groups in any amino acid, or salt forms thereof, as well as containing a non-natural amino acid such as the d-amino acid forms. The peptide compound can be PEGylated using techniques known to those skilled in the art, such as, for example, PEGylation with reagents containing the succinimidyl group, which preferentially react with primary amines present in the N-terminal region of the peptide. The peptide compound of the invention can be alkylated to any amino acid using techniques known to those skilled in the art, including, for example, the Mitsunobu reaction described in the article of Reichwein & Liskamp (Reichwein JF & Liskamp RMJ. "Site-specific N-alkylation of peptides on the solid phase", Tetrahedron Letters, Volume 39, Issue 10, 5 March 1998, Pages 1243-1246). Said article describes the introduction of any alkyl group into a specific amide function of a peptide. The peptide compound of the invention can be alkoxylated, substituted with halogens, hydroxy or other functional groups on any amino acid using techniques known to those skilled in the art, including, for example, those described in the book/publication Special Periodic Reports, Aminoacids, Peptides and Proteins: Volume 42, Royal Society of Chemistry, 2013. The peptide compound of the invention can be modified with other molecular species useful in diagnostic and/or therapeutic applications, as is the case of Biotin, using techniques known to those skilled in the art.

### Cyclic or circular peptide

In the context of the present patent application, "cyclic, cyclized or circular peptide" should be understood as a peptide that has a covalent bond between two different regions of a linear peptide molecule, through any method known in the art, particularly by the activity of enzymes. The cyclic peptide can be used instead of the linear peptide due to the fact that it is more difficult to be degraded, since its ends or areas of attack by hydrolyzing enzymes are not as exposed as in a linear peptide.

### Equivalent Dose in Humans

In the present invention, the concept of "equivalent dose in humans" is the dose at which, in humans, the same magnitude of effects observed in animals at a given dose is expected, as recommended by the *"*Guidance for Industry Estimating the Maximum Safe Starting Dose in Initial Clinical Trials for Therapeutics in Adult Healthy Volunteers" published by the U.S. Department of Health and Human Services Food and Drug Administration Center for Drug Evaluation and Research (CDER), July 2005 Pharmacology and Toxicology. In said guide, the conversion of dose observed in animals (mg/kg) to Equivalent Dose in Humans (mg/kg) implies dividing the result obtained in rats by 6.2 and the result obtained in mice by 12.3. These values are applicable to a human with a standard weight of 60 kg. For other species or for weights outside the standard weight ranges, the Human Equivalent Dose (HED) can be calculated by the formula: HED = animal dose in mg/kg x (animal weight in kg/human weight in kg)^{0.33}. Said *Guidance* considers adequate a safety range of 10 times the concentration limits tested.

In the present patent application, it is demonstrated that the use of said peptide compound provides a decrease in the levels of cytokines such as TNFα, IL-6 and/or IL-1β in mammals, with the additional advantage of being administered orally. Any therapeutic indication related to these targets can benefit from the present invention, this being particularly the case of COVID-19 and/or immune/metabolic dysfunctions associated with the effects of the Spike protein, among other conditions.

The present invention is also defined by the following clauses.

Use of the peptide compound consisting essentially of NFKF, NFKL, and/or a salt thereof, wherein one or more from said amino acids may be a d-amino acid, modified or cyclic form, may be an amidated, alkylated, alkoxylated, halogenated, hydroxylated, PEGylated version; and/or combinations thereof, for the manufacture of a preventive, curative or prophylactic medicament for immune disorders or metabolic disorders associated with elevated levels of TNFα, IL-6 and/or IL-1β in mammals.

Use of the peptide compound described above for the manufacture of a medicament for reducing ACE2 expression and/or modulating the ACE/ACE2 balance, for the treatment of hypertension, of cardiovascular or renal events, of type 2 diabetes, and for reducing the process of viral infection by SarsCov-2.

Use of the peptide compound described above for the manufacture of a preventive, curative or prophylactic medicament for diseases caused by or associated with viruses, such as influenza, coronaviruses, including COVID-19, or conditions associated with these viruses or parts thereof, in a mammal.

Use of the peptide compound described above for the manufacture of a preventive, curative or prophylactic medicament for myocarditis and/or pericarditis in a mammal.

Use of the peptide compound described above for the manufacture of a preventive, curative or prophylactic medicament for coagulopathies, thrombotic and/or cardiovascular events, or type 2 diabetes in a mammal.

Use of the peptide compound described above for the manufacture of a medicament combined with or as an adjuvant to a monoclonal antibody and/or cell therapy.

Use of the peptide compound described above for the manufacture of a medicament combined with or in place of one or more from adalimumab, infliximab, golilumab, certolizumab, sarilumab, tocilizumab, or combinations thereof.

Pharmaceutical composition modulating cytokine levels in a mammal comprising a pharmaceutically acceptable vehicle; and, as active ingredient, the compound described above.

Pharmaceutical composition for the preventive, curative or prophylactic treatment of immune disorders or metabolic disorders associated with elevated levels of TNFα, IL-6 and/or IL-1β in mammals, comprising a pharmaceutically acceptable vehicle; and, as active ingredient, the compound described above.

Pharmaceutical composition modulating ACE2 expression and/or ACE/ACE2 balance in a mammal comprising a pharmaceutically acceptable vehicle; and, as active ingredient, the compound described above.

Pharmaceutical composition for the preventive, curative or prophylactic treatment of diseases caused by or associated with viruses, including influenza, coronaviruses, including COVID-19, in a mammal, comprising a pharmaceutically acceptable vehicle; and, as active ingredient, the compound described above.

Pharmaceutical composition for the preventive, curative or prophylactic treatment of myocarditis and/or pericarditis in a mammal, comprising a pharmaceutically acceptable vehicle; and, as active ingredient, the compound described above.

Pharmaceutical composition for the preventive, curative or prophylactic treatment of hypertension, coagulopathies, thrombotic, cardiovascular and/or renal events in a mammal, comprising a pharmaceutically acceptable vehicle; and, as active ingredient, the compound described above.

Pharmaceutical composition for use in the preventive, curative or prophylactic treatment of immune disorders or metabolic disorders associated with elevated levels of TNFα, IL-6 and/or IL-1β in a mammal, comprising a pharmaceutically acceptable vehicle; and, as an active ingredient, the compound described above in the form of a tablet, gel, liquid, syrup, capsule, in oral, inhalable or patch form.

Pharmaceutical composition for reducing or eliminating side effects of antibody therapies, monoclonal antibodies, or cell therapies in a mammal, comprising a pharmaceutically acceptable vehicle; and, as an active ingredient, the compound described above in the form of a tablet, gel, liquid, syrup, capsule, in oral, inhalable or patch form.

Pharmaceutical composition for use in the preventive, curative or prophylactic treatment of diseases caused by or associated with viruses, including influenza, coronavirus, including COVID-19, in a mammal, comprising a pharmaceutically acceptable vehicle; and, as an active ingredient, the compound described above in the form of a tablet, gel, liquid, syrup, capsule, in oral, inhalable or patch form.

Pharmaceutical composition for use in the preventive, curative or prophylactic treatment of myocarditis and/or pericarditis in a mammal, comprising a pharmaceutically acceptable vehicle; and, as an active ingredient, the compound described above in the form of a tablet, gel, liquid, syrup, capsule, in oral, inhalable or patch form.

Pharmaceutical composition for use in the preventive, curative or prophylactic treatment of hypertension, coagulopathies, thrombotic, cardiovascular or renal events, or type 2 diabetes in a mammal, comprising a pharmaceutically acceptable vehicle; and, as an active ingredient, the compound described above in the form of a tablet, gel, liquid, syrup, capsule, in oral, inhalable or patch form.

A method for treating immune disorders or metabolic disorders associated with elevated levels of TNFα, IL-6 and/or IL-1β in a subject in need thereof, comprising administering to the subject a pharmaceutical composition comprising a pharmaceutically acceptable vehicle and an active ingredient consisting essentially of the compound described above.

Method for treating immune disorders or metabolic disorders associated with elevated levels of TNFα, IL-6 and/or IL-1β in a subject in need thereof, comprising administering to the subject a pharmaceutical composition comprising the peptide described above combined with a therapy comprising one or more antibodies, monoclonal antibodies or a cell therapy; or combined administration with or in place of one or more from adalimumab, infliximab, golilumab, certolizumab, sarilumab, tocilizumab, or combinations thereof.

Method for treating myocarditis and/or pericarditis in a subject in need thereof, comprising administering to the subject a pharmaceutical composition comprising a pharmaceutically acceptable vehicle and an active ingredient consisting essentially of the compound described above.

Method for treating hypertension, coagulopathies, thrombotic, cardiovascular or renal events in a subject in need thereof, comprising administering to the subject a pharmaceutical composition comprising a pharmaceutically acceptable vehicle and an active ingredient consisting essentially of the compound described above.

Method for treating diseases caused by or associated with viruses, including influenza, coronavirus, including COVID-19 in a subject in need thereof, comprising administering to the subject a pharmaceutical composition comprising a pharmaceutically acceptable vehicle and an active ingredient consisting essentially of the compound described above.

Method as described above, wherein the active ingredient is formulated in the form of a tablet, gel, liquid, syrup, capsule, inhalant or patch.

Method as indicated above, wherein the compound is administered in the dosage range from 4 to 400 µg of active ingredient per day per kg of a human patient.

Regardless of the mechanism of action, which is not the subject of this patent application, the fact that said compound has provided an important modulating action on cytokine levels in mammals clearly shows the surprising magnitude and relevance of the technical problems solved.

The pharmaceutical composition of the invention can be administered in the form of a tablet, gel, capsule, nasal or pulmonary instillation capsule, oral liquid or syrup, suppository, injectable solution, or other suitable administration forms for pharmaceutical and medical purposes.

The examples shown below are intended only to exemplify some of the different ways of implementing the invention, however, without limiting its scope.

### Examples

### Example 1. Use of the NFKF compound for the preparation of pharmaceutical composition

In this embodiment, the NFKF tetrapeptide was synthesized by chemical synthesis, using Fmoc (N-(9-fluorenyl)methoxycarbonyl) chemistry, and subsequently purified by HPLC to purity ≥95% (Proteimax Biotecnologia Ltda., São Paulo, SP), similarly to protocol previously described in Ferrante C et al., 2017; Leone S et al., 2017.

Said peptide was used in the preparation of a liquid pharmaceutical composition for oral use comprising between 2.7×10⁻⁴ Molar of said peptide and a pharmaceutically acceptable vehicle. In this embodiment, said vehicle is saline, the pharmaceutical composition being an oral solution. Said composition was used for in vivo oral administration to mammals as described below.

In other embodiments, the pharmaceutical composition is in the form of a tablet, gel, oral liquid or syrup, capsule, suppository, injectable solution or inhalable or patch forms, optionally comprising other active ingredients.

### Example 2. Pharmaceutical composition comprising NFKF to reduce TNFα levels

In the present example, experiments were conducted to demonstrate the ability of the composition of the invention to substantially reduce TNFα levels in vivo. As an experimental model, experimental acute encephalomyelitis (EAE) induction was used, as described in the literature (Basso AS et al., 2008; Santos NBD et al., 2019).

The pharmaceutical composition as described in example 1 was administered to female wild-type C57BL/6J mice. All animal experiments were conducted double-blind until completion of the experiment (Sjoberg EA, 2017). The animals had free access to food and water in a temperature-controlled environment (Sjoberg EA, 2017) between 22 and 23 °C and with a 12h light/dark cycle, the lights being turned on at 6:30 am. The animals were maintained and used in accordance with the recommendations of the National Council for the Control of Animal Experimentation *"Conselho National para Controle de Experimentação Animal"* (CONCEA), following international standards for animal care and maintenance.

EAE induction was performed as described (Basso AS et al., 2008; Santos NBD et al., 2019). Briefly, 24h before immunization, 20 µL of blood was collected from the animal's tail and diluted in 1 mL of saline phosphate buffer, pH 7.5 (PBS), and immediately read on a BC2800 vet analyzer (BioBrasil, São Paulo, SP, Brazil) to quantify leukocytes. On the day of immunization (day 0), under inhaled anesthesia (isoflurane 1.5% in oxygen), WT females received a subcutaneous injection of 200 µg of MOG (myelin oligodendrocyte glycoprotein peptide 35-55; Proteimax Biotecnologia Ltda., São Paulo, SP), emulsified in 100 µL of complete Freund's adjuvant containing 4 mg/mL of thermoinactivated *Mycobacterium tuberculosis.* In addition, 300 ng of *Bordetella pertussis* toxin was administered intraperitoneally (I.P.) twice, at 0 and 48h after immunization. Control animals were treated with vehicle (complete Freund's adjuvant, *subcutaneous;* pertussis toxin, *intraperitoneal*). NFKF peptide compound (0.5 mg/kg *per day*) or 0.9% NaCl/saline (Salt) was administered orally by *gavage* starting on day 1. Animals (n = 9-10) were evaluated daily based on clinical scores.

In this dosage regimen in mice, when converting to the Equivalent Dosage in Humans is applied, a therapeutic method in which the compound is administered in the dosage range from 4 to 400 µg of the active ingredient per day per kg of a patient human is obtained.

Fig. 1 shows the results of tests with female C57BL6 animals (mice), 8 to 10 weeks old, which were submitted to an induction protocol of Experimental Autoimmune Encephalomyelitis (EAE). On the y-axis, TNFα concentrations are shown in arbitrary units, under different test conditions as indicated, in which 10 µL of plasma was used for quantification by ELISA according to the manufacturer's specifications (R&D Systems). The results in figure 1 show a substantial and significant reduction in the concentration of TNFα upon the administration of NFKF to the animals.

TNFα is an inflammatory cytokine involved in immune function and has been reported to also be related to metabolic disorders and/or coagulopathies. In this context, a number of recently published scientific articles shed light on the role of the coronavirus Spike protein in activating the NF-κB and/or TNFα pathway.

Now, it is known that COVID-19 involves overactivation of the NF-κB pathway (Hariharan A, Hakeem AR, Radhakrishnan S, Reddy MS, Rela M. The Role and Therapeutic Potential of NF-kappa-B Pathway in Severe COVID-19 Patients. Inflammopharmacology. 2021 Feb;29(1):91-100. doi: 10.1007/s10787-020-00773-9. 38. Kircheis R, Haasbach E, Lueftenegger D, Heyken WT, Ocker M, Planz O. NF-κB Pathway as a Potential Target for Treatment of Critical Stage COVID-19 Patients. Front Immunol. 2020 Dec 10;11:598444. doi: 10.3389/fimmu.2020.598444). In this context, the literature has shown that the SarsCov-2 Spike protein potentially induces the expression of the NF-κB pathway and inflammatory cytokines, including IL-6, IL-1β, TNFα, in addition to CXCL1, CXCL2, and CCL2 (but not IFNs) in human and mouse macrophages (Khan S, Shafiei MS, Longoria C, Schoggins J, Savani RC, Zaki H. SARS-CoV-2 spike protein induces inflammation via TLR2-dependent activation of the NF-κB pathway. bioRxiv [Preprint]. 2021 Mar 17:2021.03.16.435700. doi: 10.1101/2021.03.16.435700). Furthermore, the involvement of the NF-κB or TNFα pathway in coagulopathies is described in the literature, so that having an oral therapeutic approach that modulates TNFα concentration is potentially useful in the context of complications associated with Spike protein or COVID-19.

### Example 3. Pharmaceutical composition comprising NFKF to reduce IL-6 levels

In the present example, experiments were conducted to demonstrate the ability of the composition of the invention to substantially reduce in vivo levels of the cytokine IL-6. As an experimental model, experimental acute encephalomyelitis (EAE) induction was used, as described in the literature (Basso AS et al., 2008; Santos NBD et al., 2019).

The pharmaceutical composition as described in example 1 was administered to female wild-type C57BL/6J mice. All animal experiments were conducted double-blind until completion of the experiment (Sjoberg EA, 2017). The animals had free access to food and water in a temperature-controlled environment (Sjoberg EA, 2017) between 22 and 23 °C and with a 12h light/dark cycle, the lights being turned on at 6:30 am. The animals were maintained and used in accordance with the recommendations of the National Council for the Control of Animal Experimentation *"Conselho National para Controle de Experimentação Animal"* (CONCEA), following international standards for animal care and maintenance.

EAE induction was performed as described (Basso AS et al., 2008; Santos NBD et al., 2019). Briefly, 24h before immunization, 20 µL of blood was collected from the animal's tail and diluted in 1 mL of saline phosphate buffer, pH 7.5 (PBS), and immediately read on a BC2800 vet analyzer (BioBrasil, São Paulo, SP, Brazil) to quantify leukocytes. On the day of immunization (day 0), under inhaled anesthesia (isoflurane 1.5% in oxygen), WT females received a subcutaneous injection of 200 µg of MOG (myelin oligodendrocyte glycoprotein peptide 35-55; Proteimax Biotecnologia Ltda., São Paulo, SP), emulsified in 100 µL of complete Freund's adjuvant containing 4 mg/mL of thermoinactivated *Mycobacterium tuberculosis.* In addition, 300 ng of *Bordetella pertussis* toxin was administered intraperitoneally (I.P.) twice, at 0 and 48h after immunization. Control animals were treated with vehicle (complete Freund's adjuvant, *subcutaneous;* pertussis toxin, *intraperitoneal*). NFKF peptide compound (0.5 mg/kg *per day*) or 0.9% NaCl/saline (Sal) was administered orally by *gavage* starting on day 1. Animals (n = 9-10) were evaluated daily based on clinical scores.

Fig. 2 shows the results of tests for the evaluation of the level of IL-6 and its modulation in an established experimental model, in which female C57BL6 animals (mice), from 8 to 10 weeks old, were submitted to an Experimental Autoimmune Encephalomyelitis (EAE) induction protocol. Eight days after EAE induction, the animals were anesthetized with ketamine (90 mg/kg) and xylazine (10 mg/kg). After dissection of the spleen and inguinal lymph nodes, leukocytes were extracted. Cells were stimulated with Anti-CD3 (1 µg/mL) and MOG (100 pg/mL) with or without said peptide compound in the NFKF embodiment (100 µg/mL) for 72 hours. Then, 50 µL of medium were used for IL-6 cytokine quantification by ELISA. In A, IL-6 is shown by quantification by ELISA on spleen leukocyte cells. In B, it is IL-6 as measured by ELISA in leukocyte cells from inguinal lymph nodes. Results are expressed as mean ± standard deviation. Statistical analyzes were performed using the One-way ANOVA test, n = 4.

As the clinical scores observed in the experiment were clearly better in the NFKF-treated group, another series of experiments was conducted to assess the impact on cytokine levels in animals from both groups. Leukocyte samples from the spleen and inguinal lymph nodes were analyzed to quantify the extracted levels of IL-6. After extraction with hypotonic buffer, cells were analyzed with an Acuri flow cytometer (BD Biosciences, NJ, USA) for total cell count. Next, one million spleen and inguinal lymph node cells from animals with EAE were plated in 96-well ELISA plates, stimulated with anti-CD3 (1 µg/mL) and MOG (100 µg/mL), and treated with saline (controls) or NFKF (100 µg/mL) for 72 hours. Then, 50 µL of medium were used for IL-6 cytokine quantification by Elisa assays following the manufacturer's specifications (R&D Systems).

The results in figure 2 show that the administration of NFKF significantly and substantially reduced the levels of IL-6 in the spleen leukocytes of the animals in which EAE was induced, either with or without stimulation with anti-CD3/MOG.

### Example 4. Pharmaceutical composition comprising NFKF for reducing levels of IL-1β

In the present example, experiments were conducted to demonstrate the ability of the composition of the invention to substantially reduce in vivo levels of the cytokine IL-1β. As an experimental model, experimental acute encephalomyelitis (EAE) induction was used, as described in the literature (Basso AS et al., 2008; Santos NBD et al., 2019).

The pharmaceutical composition as described in example 1 was administered to female wild-type C57BL/6J mice. All animal experiments were conducted double-blind until completion of the experiment (Sjoberg EA, 2017). Animals had free access to food and water in a temperature-controlled environment (Sjoberg EA, 2017) between 22 and 23 °C and with a 12h light/dark cycle, the lights being turned on at 6:30 am. The animals were maintained and used in accordance with the recommendations of the National Council for the Control of Animal Experimentation *"Conselho National de Controle de Experimentação Animal"* (CONCEA), following international standards for animal care and maintenance.

EAE induction was performed as described (Basso AS et al., 2008; Santos NBD et al., 2019). Briefly, 24h before immunization, 20 µL of blood was collected from the animal's tail and diluted in 1 mL of saline phosphate buffer, pH 7.5 (PBS), and immediately read on a BC2800 vet analyzer (BioBrasil, São Paulo, SP, Brazil) to quantify leukocytes. On the day of immunization (day 0), under inhaled anesthesia (isoflurane 1.5% in oxygen), WT females received a subcutaneous injection of 200 µg of MOG (myelin oligodendrocyte glycoprotein peptide 35-55; Proteimax Biotecnologia Ltda., São Paulo, SP), emulsified in 100 µL of complete Freund's adjuvant containing 4 mg/mL of thermoinactivated *Mycobacterium tuberculosis.* In addition, 300 ng of *Bordetella pertussis* toxin was administered intraperitoneally (I.P.) twice, at 0 and 48h after immunization. Control animals were treated with vehicle (complete Freund's adjuvant, *subcutaneous;* pertussis toxin, *intraperitoneal).* NFKF peptide compound (0.5 mg/kg *per day)* or 0.9% NaCl/saline (Sal) was administered orally by *gavage* starting on day 1. Animals (n = 9-10) were evaluated daily based on clinical scores.

Fig. 3 shows the results of tests for the evaluation of the level of IL-1β and its modulation in an established experimental model, in which female C57BL6 animals (mice), from 8 to 10 weeks old, were submitted to an Experimental Autoimmune Encephalomyelitis (EAE) induction protocol. Eight days after EAE induction, the animals were anesthetized with ketamine (90 mg/kg) and xylazine (10 mg/kg). After dissection of the spleen and inguinal lymph nodes, leukocytes were extracted. Cells were stimulated with Anti-CD3 (1 µg/mL) and MOG (100 µg/mL) with or without said peptide compound in the NFKF embodiment (100 µg/mL) for 72 hours. Then, 50 µL of medium were used to IL-1β cytokine quantification by ELISA. In A, IL-1β is shown by quantification by ELISA in spleen leukocyte cells. In B, it is IL-1β as measured by ELISA in leukocyte cells from inguinal lymph nodes. Results are expressed as mean ± standard deviation. Statistical analyzes were performed using the One-way ANOVA test, n = 4.

As the clinical scores observed in the experiment were clearly better in the NFKF-treated group, another series of experiments was conducted to assess the impact on cytokine levels in animals from both groups. Samples of leukocytes from the spleen and inguinal lymph nodes were analyzed to quantify the extracted levels of IL-1β. After extraction with hypotonic buffer, cells were analyzed with an Acuri flow cytometer (BD Biosciences, NJ, USA) for total cell count. Next, one million spleen and inguinal lymph node cells from animals with EAE were plated in 96-well ELISA plates, stimulated with anti-CD3 (1 µg/mL) and MOG (100 µg/mL), and treated with saline (controls) or NFKF (100 µg/mL) for 72 hours. Then, 50 µL of medium were used for IL-1β cytokine quantification by Elisa assays following the manufacturer's specifications (R&D Systems).

The results in figure 3 show that the administration of NFKF significantly and substantially reduced the levels of IL-1β in the spleen leukocytes of the animals in which EAE was induced, either with or without stimulation with anti-CD3/MOG. Similarly, in the inguinal lymph nodes there was a significant and substantial reduction in IL-1β levels upon administration of NFKF, regardless of the use or not of stimulation with anti-CD3/MOG.

Previous reports suggest that IL-1β leads to neuroinflammation in a mouse model of EAE (Levesque SA et al., 2016). This experiment showed that NFKF largely reduced IL-1β levels in mice in the EAE model, whether or not they were stimulated with anti-CD3/MOG. The magnitude of IL-1β reduction, both in the spleen and inguinal lymph nodes, either with or without stimulation with an inflammatory agent (anti-CD3/MOG), is surprising and indicates important therapeutic applications.

In addition to neurotransmitter excitotoxicity, IL-1β is known to play an important role in EAE. Clinical EAE is significantly attenuated in IL-1 receptor-deficient and IL-1β-deficient mice, with IL-1β found in blood, cerebrospinal fluid, and CNS lesions in patients with Multiple Sclerosis/MS.

The results presented in these examples are proof of concept of the reduction of IL-1β cytokine levels, indicating that the invention is a therapeutic alternative for the treatment of patients with type 2 diabetes. The present invention, by providing a substantial reduction in IL-1β levels, is a therapeutic alternative for the preventive, prophylactic or curative treatment of type 2 diabetes.

It is important to note that recently anecdotal cases have been reported of people who had cases of inflammation of the heart, peripheral nervous system, brain and spinal cord, possibly associated with mRNA vaccines, as published in the *Journal of Neurology* on 03-Sep-2021 (Journal of Neurology https://doi.org/10.1007/s00415-021-10780-7; COVID-19 mRNA vaccination leading to CNS inflammation: a case series). In the specific case of patients with Multiple Sclerosis (MS), neurological symptoms (and nuclear magnetic resonance imaging findings) consistent with active demyelination of the optic nerve, brain, or spinal cord have been reported. Therefore, experimental models of MS can be very useful in the context of evaluating therapeutic approaches for the treatment of COVID-19 or side effects associated with Spike protein, virus, or vaccine vectors and/or vaccine protocol.

The significant in vivo reduction of TNFα, IL-6 and/or IL-1β levels is also proof of concept of the applicability of the invention in treatment methods for the conditions indicated in the present specification. It is also a proof of concept for its usefulness in the manufacture of a combined medicament with or as an adjunct to monoclonal antibody therapies or cell therapies, and also as a medicament combined with or in place of one or more from adalimumab, infliximab, golilumab, certolizumab, sarilumab, tocilizumab, or combinations thereof.

The test results shown in examples 2, 3 and 4 above also support an important role in the regulation of the complex system of angiotensins ACE and ACE2, which has important impacts in several conditions, such as hypertension, various cardiovascular and renal events, as well as in the COVID-19, since ACE2 has a recognized role in the infection process by the respective virus.

As the administration of the composition of the invention led to a demonstrated and concomitant decrease in the levels of TNFα and IL-6, this is a proof of concept of a new therapeutic approach to reduce the expression of ACE2, since this expression is increased by TNFα alone and IL-6 alone. Reducing ACE2 expression has a variety of therapeutic applications, including the treatment of hypertension, cardiovascular or renal events, as well as potentially limiting the progression of SarsCov-2 infection.

The reduction of the positive feedback effect (increased IL-6 and IL1-β expression when ACE2 levels are high and increased ACE2 expression when IL-6 and TNFα levels increase), which can lead to the cytokine storm and is related to the severity of the disease COVID-19 is another therapeutic potential demonstrated in the present invention. Consequently, the present invention provides a therapeutic alternative that can contribute to the fight against COVID-19 by more than one mechanism: helping to minimize or prevent viral replication by reducing the expression of ACE2; and contribute to minimize the severe disease associated with the cytokine storm.

### Example 5. No detection of undesirable central effects upon in vivo administration of composition comprising NFKF

In vivo tests were performed to evaluate possible undesirable central effect, as known for the closest molecular entities, hemopressin and RVD-hemopressin.

Fig. 4 shows the results of in vivo tests to assess possible undesirable effects on the central nervous system, using the elevated plus maze (EPM) test, a test to measure anxiety in laboratory animals and to assess anxiolytic or anxiogenic compounds. The results demonstrate that the administration of NFKF does not lead to such undesirable central effects. The results in figure 4 demonstrate that the administration of NFKF does not lead to central effects. Saline solution (Veh) was used as a negative control, the compound Win 55212-2 (WIN), a known cannabinoid 1 receptor agonist, as a positive control and NFKF at the indicated concentrations, all administered orally by gavage in C57B6 mice, in a total of 7 animals. In A) the temperature variation is shown in °C (delta °C); in B) the time of catalepsy in seconds after administration is shown; in C) the walking distance in meters is shown in the open field walking test; in D) the % of MPE (maximal possible effect) in the hot plate test is shown; in E) the % of entries in the open arms in the EPM (elevated plus maze) test is shown; in F) the % of time spent in the open arms in the EPM test is shown.

The fact that NFKF treatment does not induce depressive/anxiogenic behavior shows the specificity of action in the central nervous system. Reaching specific nuclei that confer the neuro-protective capacity of NFKF (object of the co-pending international patent application published as WO 2018209415) and not causing a generalized modulation that can generate an imbalance, manifesting itself with behavioral changes.

The results in figure 4 show that the administration of the composition containing NFKF provided surprisingly contrasting results when compared to the known central effects of hemopressin and RVD-hemopressin, the latter inducing anxiolytic and antidepressant behavior and the former inducing anxiogenic and depressive behavior, in addition to reducing of basal monoamine levels in the prefrontal cortex and increased expression of genes involved in its catabolism. The administration of NFKF did not change any type of behavior of the animals in the tests conducted, as evidenced in panels A-F of figure 4. The data show that the administration of the compound of the invention does not induce an undesirable central effect, that is, it does not cause the known central effects in their closest congeners. This important technical advantage enables, in practice, its use in a wider range of conditions.

The inventive concept now disclosed and exemplified in one or more manners was treated as a trade secret and was not previously revealed until the filing of this patent application or its priority. This trade secret is the depositor's intangible asset. Any future publication of the patent application does not, in itself, constitute authorization for use by third parties, serving only as: (i) making third parties aware of the existence of said trade secret on the date of filing; (ii) unequivocal indication of its holder; and (iii) encouraging the development of new improvements based on the concept just revealed, to avoid reinvestment in the development of the same asset already owned by the depositor.

It is immediately warned that any commercial use requires authorization from the holder and that unauthorized use entails sanctions provided for by law. In this context, it is clarified that from the disclosure of the present inventive concept, those skilled in the art may consider other forms of embodiments of the invention that are not identical to those merely exemplified above, but that in the event of intention of commercial use such forms may be considered as being within the scope of the attached claims.

## Claims

1. Use of the peptide compound NFKF, NFKL, and/or modified, cyclic forms thereof, amidated, alkylated, alkoxylated, halogenated, hydroxylated, PEGylated versions, modified with other functional groups, as well as with a non-natural amino acid such as d-amino acid forms, salts thereof; and/or combinations thereof **characterized in that** it is for the manufacture of a medicament useful in mammals for:
- reducing the levels of tumor necrosis factor alpha (TNFα) cytokines, interleukin 6 (IL-6) and/or interleukin 1 beta (IL-1β);
- preventive, curative, or prophylactic treatment of immune disorders or metabolic disorders associated with elevated levels of TNFα, IL-6 and/or IL-1β;
- reducing ACE2 expression, modulate the renin-angiotensin system, and/or modulate ACE/ACE2 balance;
- reducing or eliminating side effects of antibody therapies, monoclonal antibodies or cellular therapies;
- combination treatment with or in place of one or more from adalimumab, infliximab, golilumab, certolizumab, sarilumab, tocilizumab, or combinations thereof;
- preventive, curative, or prophylactic treatment of myocarditis and/or pericarditis;
- preventive, curative, or prophylactic treatment of hypertension, coagulopathies, thrombotic, cardiovascular or renal events, type 2 diabetes; and/or
- the preventive, curative or prophylactic treatment of infections or diseases caused by or associated with viruses, including influenza, and coronaviruses, including COVID-19, or conditions associated with these viruses or parts thereof.

2. Use, according to claim 1, **characterized in that** it is for the manufacture of a preventive, curative or prophylactic medicament for myocarditis and/or pericarditis.

3. Use, according to claim 1, **characterized in that** it is for the manufacture of a preventive, curative or prophylactic medicament for hypertension, coagulopathies, thrombotic, cardiovascular or renal events, type 2 diabetes.

4. Use, according to claim 1, **characterized in that** it is for the manufacture of a preventive, curative or prophylactic medicament for diseases caused by or associated with viruses, influenza, coronavirus, COVID-19, or conditions associated with these viruses or parts thereof.

5. Pharmaceutical composition for the preventive, curative or prophylactic treatment of immune disorders or metabolic disorders associated with elevated levels of TNFα, IL-6 and/or IL-1β in a mammal **characterized in that** it comprises a pharmaceutically acceptable vehicle; and, as active ingredient, the peptide compound NFKF, NFKL, and/or modified, cyclic forms thereof, amidated, alkylated, alkoxylated, halogenated, hydroxylated, PEGylated versions, modified with other functional groups, as well as with a non-natural amino acid such as the d-amino acid forms, salts thereof; and/or combinations thereof.

6. Pharmaceutical composition for the preventive, curative or prophylactic treatment of hypertension, myocarditis, pericarditis, coagulopathies, thrombotic, cardiovascular and/or renal events, type 2 diabetes in a mammal **characterized in that** it comprises a pharmaceutically acceptable vehicle; and, as active ingredient, the peptide compound NFKF, NFKL, and/or modified, cyclic forms thereof, amidated, alkylated, alkoxylated, halogenated, hydroxylated, PEGylated versions, modified with other functional groups, as well as with a non-natural amino acid such as the d-amino acid forms, salts thereof; and/or combinations thereof.

7. Pharmaceutical composition for the preventive, curative or prophylactic treatment of infections or diseases caused by or associated with viruses, influenza, coronavirus, of COVID-19, or conditions associated with these viruses or parts thereof in a mammal **characterized in that** it comprises a pharmaceutically acceptable vehicle; and, as active ingredient, the peptide compound NFKF, NFKL, and/or modified, cyclic forms thereof, amidated, alkylated, alkoxylated, halogenated, hydroxylated, PEGylated versions, modified with other functional groups, as well as with a non-natural amino acid such as the d-amino acid forms, salts thereof; and/or combinations thereof.

8. Pharmaceutical composition for reducing or eliminating the side effects of antibody therapies, monoclonal antibodies or cell therapies in a mammal **characterized in that** it comprises a pharmaceutically acceptable vehicle; and, as active ingredient, the peptide compound NFKF, NFKL, and/or modified, cyclic forms thereof, amidated, alkylated, alkoxylated, halogenated, hydroxylated, PEGylated versions, modified with other functional groups, as well as with a non-natural amino acid such as the d-amino acid forms, salts thereof; and/or combinations thereof.

9. Pharmaceutical composition according to any one of claims 5 to 8, **characterized in that** it is presented in the form of a tablet, gel, oral liquid or syrup, capsule, suppository, injectable solution, inhalable or patch form.

10. Pharmaceutical composition for use in the preventive, curative or prophylactic treatment of immune disorders or metabolic disorders associated with elevated levels of TNFα, IL-6 and/or IL-1β in a mammal, comprising a pharmaceutically acceptable vehicle; and, as active ingredient, the peptide compound NFKF, NFKL, and/or modified, cyclic forms thereof, amidated, alkylated, alkoxylated, halogenated, hydroxylated, PEGylated versions, modified with other functional groups, as well as with a non-natural amino acid such as the d-amino acid forms, salts thereof; and/or combinations thereof **characterized in that** it is presented in the form of a tablet, gel, liquid, syrup, capsule, oral, inhalable or patch form.

11. Pharmaceutical composition for use in the preventive, curative or prophylactic treatment of hypertension, myocarditis, pericarditis, coagulopathies, thrombotic, cardiovascular and/or renal events, or type 2 diabetes in a mammal, comprising a pharmaceutically acceptable vehicle; and, as active ingredient, the peptide compound NFKF, NFKL, and/or modified, cyclic forms thereof, amidated, alkylated, alkoxylated, halogenated, hydroxylated, PEGylated versions, modified with other functional groups, as well as with a non-natural amino acid such as the d-amino acid forms, salts thereof; and/or combinations thereof **characterized in that it** is presented in the form of a tablet, gel, liquid, syrup, capsule, oral, inhalable or patch form.

12. Pharmaceutical composition for use in the preventive, curative or prophylactic treatment of diseases caused by or associated with viruses, including influenza, and coronaviruses, including COVID-19, or conditions associated with these viruses or parts thereof in a mammal, comprising a pharmaceutically acceptable vehicle; and, as active ingredient, the peptide compound NFKF, NFKL, and/or modified, cyclic forms thereof, amidated, alkylated, alkoxylated, halogenated, hydroxylated, PEGylated versions, modified with other functional groups, as well as with a non-natural amino acid such as the d-amino acid forms, salts thereof; and/or combinations thereof **characterized in that** it is presented in the form of a tablet, gel, liquid, syrup, capsule, oral, inhalable or patch form.

13. Method for treating immune disorders or metabolic disorders associated with elevated levels of TNFα, IL-6 and/or IL-1β in a subject in need thereof, **characterized in that** it comprises administering to the subject a pharmaceutical composition comprising a pharmaceutically acceptable vehicle and an active ingredient consisting essentially of NFKF, NFKL, and/or a salt thereof, wherein one or more from said amino acids may be a d-amino acid, modified or cyclic form, may be an amidated, alkylated, alkoxylated, halogenated, hydroxylated, PEGylated version; and/or combinations thereof.

14. Method for treating hypertension, myocarditis, pericarditis, coagulopathies, thrombotic, cardiovascular and/or renal events, or type 2 diabetes in a subject in need thereof, **characterized in that** it comprises administration to the subject of a pharmaceutical composition comprising a pharmaceutically acceptable vehicle and an active ingredient essentially consisting of NFKF, NFKL, and/or a salt thereof, wherein one or more from said amino acids may be a d-amino acid, modified or cyclic form, may be an amidated, alkylated, alkoxylated, halogenated, hydroxylated, PEGylated version; and/or combinations thereof.

15. Method for the treatment of diseases caused by or associated with viruses, including influenza, coronavirus, including COVID-19, or conditions associated with these viruses or parts thereof, in a subject in need thereof, **characterized in that** it comprises administration to the subject of a pharmaceutical composition comprising a pharmaceutically acceptable vehicle and an active ingredient consisting essentially of NFKF, NFKL, and/or a salt thereof, wherein one or more from said amino acids may be a d-amino acid, modified or cyclic form, may be an amidated, alkylated, alkoxylated, halogenated, hydroxylated, PEGylated version; and/or combinations thereof.

16. Method according to any one of claims 13 to 15, **characterized in that** said active ingredient is formulated in the form of a tablet, gel, liquid, syrup, capsule, inhalant or patch.

17. Method according to any one of claims 13 to 16, **characterized in that** it comprises: combined administration with a therapy with one or more antibodies, monoclonal antibodies and/or cell therapy; or combined administration with or in place of one or more from adalimumab, infliximab, golilumab, certolizumab, sarilumab, tocilizumab, or combinations thereof.
